# EUROPÄISCHE PATENTSCHRIFT

(11) **EP 1 053 747 B1**
(45) Veröffentlichungstag und Bekanntmachung des Hinweises auf die Patenterteilung: **30.06.2004**
(21) Anmeldenummer: 00110426.4
(22) Anmeldetag: 16.05.2000
(51) Int. Cl.: A61K 9/70, A61K 6/00, A61K 49/04, A61K 33/38, A61K 33/34, A61P 31/04

(54) **Mittel zur Behandlung lokaler parodontaler Infektionen**
Composition for treating topical periodontal infection
Composition de traitement des infections topiques parodontales

(30) Priorität: 18.05.1999 DE 29908415 U
(43) Veröffentlichungstag der Anmeldung: 22.11.2000
(73) Patentinhaber: Carl, Wolfgang, Dr. med. dent., 66386 St. Ingbert (DE)
(72) Erfinder: Carl, Wolfgang, Dr. med. dent., 66386 St. Ingbert (DE)
(74) Vertreter: Vièl, Christof, Dipl.-Ing.

(56) Entgegenhaltungen:
- EP-A- 0 140 766
- WO-A-90/00048
- WO-A-99/08691
- US-A- 5 186 936
- PATENT ABSTRACTS OF JAPAN vol. 013, no. 205 (C-595), 15. Mai 1989 (1989-05-15) & JP 01 025734 A (YASUYUKI SUGANO), 27. Januar 1989 (1989-01-27)

## Beschreibung

Die Erfindung betrifft ein Mittel zur Behandlung lokaler parodontaler Infektionen, bestehend aus einem mit einer pharmakologisch aktiven Substanz getränkten Faden, der in dem infizierten Bereich befestigbar ist und aus dem die Substanz langsam freisetzbar ist.

Behandlungsbedürftige Parodontalerkrankungen treten bei 75 % der Erwachsenen in Deutschland auf. Sie werden durch gramnegative Keime und bewegliche Stäbchen, wie z.B. Actinobacillus actinomycetemcomitans, Porphyromonas gingivalis und Prevotella intermedia hervorgerufen, deren Stoffwechselprodukte Entzündungsvorgänge hervorrufen. Deshalb ist die Elimination bzw. Reduktion dieser Mikroorganismen bei der Prävention und der Therapie von Parodontitis von großer Bedeutung.

Neben der mechanischen Reinigung wird dies durch den Einsatz antimikrobieller Wirkstoffe erreicht. Bei der mechanischen Reinigung werden die Keime nicht immer ausreichend eliminiert, da nicht alle Wurzeloberflächen einer mechanischen Reinigung zugänglich sind und häufig Erreger die umgebenden Gewebe kolonisieren und nicht mit einfachen mechanischen Maßnahmen entfernt werden können. Daher erfolgt in der Regel eine Kombination von mechanischer Reinigung und lokalem Antibiotikaeinsatz.

Allerdings muß das eingesetzte Antibiotikum gegen alle wichtigen Erreger wirksam sein, am Wirkort eine ausreichend hohe Konzentration über einen ausreichend langen Zeitraum gewährleistet sein und die Freisetzung des Antibiotikums direkt an dem Infektionsherd erfolgen.

Hierfür wird in der EP 0 429 224 A1 vorgeschlagen, ein Antibiotika freisetzendes Gel in die entzündeten Zahnfleischtaschen einzubringen. Die EP 0 429 225 A1 und die EP 0 430 474 A1 schlagen weitere Verbindungen zum Freisetzen von Antibiotika in der Zahnfleischtasche vor. Ein sich bei pH-Werten von 4,0 und mehr in wässrigem Milieu auflösendes Polymer zum Freisetzen therapeutisch wirksamer Substanzen wird in der EP 0 241 179 A1 beschrieben.

Die DE 36 26 692 C1 beschreibt eine Vorrichtung zur Applikation von Medikamenten im Interdentalraum, die zwei weichelastische Schilde aufweist, die durch ein Zugorgan miteinander verbunden sind. Diese Schilde stützen sich an den angrenzenden Zähnen und am Zahnfleisch ab, während sie von dem Zugorgan gegeneinander gezogen werden. Dadurch wird der Interdentalraum weitgehend abgeschlossen. Mit einer Applikationskanüle kann ein Medikament oder eine Spülflüssigkeit in den Interdentalraum eingeführt werden. Die Vorrichtung ermöglicht eine lang andauernde Einwirkung des Medikamentes auf das Zahnfleisch.

Unter der Bezeichnung Actisite® vertreibt die Wybert GmbH, 79539 Lörrach einen Faden aus Poly(ethylen, vinylacetat) 60:40, in dem 25 Gew.-% Tetracyclin-HCl homogen verteilt sind. Der Faden wird in schichtweise zur Füllung der zu behandelnden Zahnfleischtaschen verwendet, mit einem Gewebekleber fixiert und setzt während 10 Tagen eine ausreichende Menge an Tetracyclin frei, daß in der Umgebung kein oraler Mikroorganismus überleben kann. Nach 10 Tagen wird der Faden entfernt.

Um eine optimale Wirkung zu erzielen, muß die Zahnfleischtasche vollständig gefüllt werden, darf jedoch auch nicht überfüllt werden, da sonst der Faden sich lockern und herausfallen kann. Dies ist nur schwierig zu kontrollieren. Da der Faden lichtempfindlich ist und bei Lichteinwirkung zerfällt, kann ein Herausfallen eines Fadens unbemerkt bleiben, so daß die Gefahr einer bakteriellen Wiederbesiedlung der behandelten Zahnfleischtaschen einer Kieferhälfte besteht.

Aus der US 5,186,936 A ist ein biokompatibler Polymerträger bekannt, in dem ein Metronidazolester enthalten ist, um durch kontrollierte kontinuierliche Freisetzung zumindest ein Bakterienwachstum verhindert werden kann. Der Polymerträger kann in Fadenform vorliegen und er kann zusätzlich mit einem Röntgenkontrastmittel, wie Bariumsulfat oder Fluorescein, versehen werden.

Aufgabe der Erfindung ist es somit, ein Mittel zur Behandlung lokaler parodontaler Infektionen gemäß dem Oberbegriff zu schaffen, das die beschriebenen Nachteile vermeidet.

Diese Aufgabe wird erfindungsgemäß dadurch gelöst, daß der Faden als Röntgenkontrastmittel einen Metallfaden, insbesondere einen Kupfer- oder Silberfaden, enthält.

Hierdurch wird es möglich, zur Kontrolle der ordnungsgemäßen Befüllung der Zahnfleischtasche eine Röntgenaufnahme zu machen. Ebenso kann auf diese Weise überprüft werden, ob alle Fäden noch vorhanden sind. Ein Metallfaden kann leicht auf dem Röntgenbild erkannt werden. Kupfer und Silber wirken zudem bakterizid.

Vorteilhaft ist hierbei, daß der Metallfaden im Zentrum des Fadens angeordnet und von die Substanz freisetzendem Gewebe umgeben ist.

Eine Weiterbildung der Erfindung besteht darin, daß zwischen dem Metallfaden und dem Gewebe eine isolierende Schicht angeordnet ist.

Es liegt im Rahmen der Erfindung, daß das Gewebe aus Poly(ethylen, vinylacetat) besteht.

Ausbildungen der Erfindung bestehen darin, daß die pharmakologisch aktive Substanz ein Antibiotikum, ein entzündungshemmender oder ein sauerstoffabspaltender Stoff ist.

Hierbei ist es zweckmäßig, daß das Antibiotikum Tetracyclin oder Metronidazol ist.

Die Vorteile der Erfindung bestehen im wesentlichen darin, daß auf einfache Weise eine Kontrolle der ordnungsgemäßen Einbringung bzw. des Verbleibes des Fadens ermöglicht wird. Außerdem kann durch die versteifende Wirkung der Metalleinlage auf die (für den Patienten unangenehme) Fixation des Fadens mittels Gewebekleber verzichtet werden.

Im folgenden werden Ausführungsbeispiele der Erfindung anhand von Zeichnungen beschrieben.

Es zeigen
- Fig. 1: ein erfindungsgemäßes Mittel zur Behandlung lokaler parodontaler Infektionen,
- Fig. 2: das Einbringen des Mittels in eine Zahnfleischtasche.

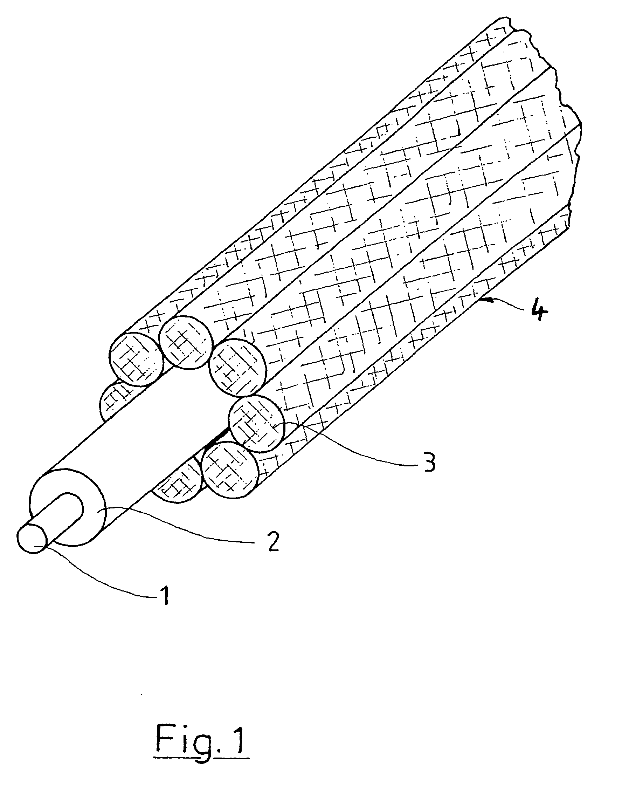

Der in Fig. 1 dargestellte Faden 4 besteht aus einem Metallkern 1, der mit einer Kunststoffisolierung 2 umgeben ist. Um diese Kunststoffisolierung 2 herum ist ein Gewebe 3 angeordnet, das beispielsweise wie bei bekannten Fäden aus Poly(vinylacetet, ethylen) 60:40 bestehen kann. In diesem Gewebe 3 ist eine pharmakologisch aktive Substanz, beispielsweise ein Antibiotikum (z.B. 25 Gew.-% Tetracyclin-HCl oder Metronidazol), homogen verteilt.

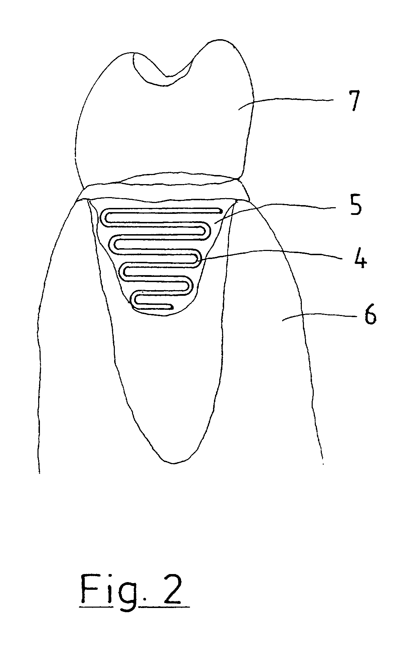

Fig. 2 zeigt das Einbringen des Fadens 4 in eine Tasche 5 im Zahnfleisch 6 eines Zahnes 7. Der Faden 4 wird mäanderförmig in die Tasche 5 eingebracht, bis diese gänzlich gefüllt ist und (optional) mit einem Gewebekleber fixiert. Pro Tasche 5 werden zwischen 4 und 15 cm Faden 4 appliziert. Durch die langsame Freisetzung des Antibiotikums aus dem Faden wird einerseits eine Antibiotikum-Konzentration in der Sulkusflüssigkeit erreicht, in der kein oraler Mikroorganismus überleben kann, andererseits wird bei einer Behandlung auch mehrerer Zähne nur eine Serumkonzentration unter der Nachweisgrenze von 0,1 µg/ml erreicht.

Nach 10 Tagen wird der Faden 4 aus der Tasche 5 entfernt und gegebenenfalls die andere Kieferhälfte behandelt.

## Patentansprüche

1. Mittel zur Behandlung lokaler parodontaler Infektionen, bestehend aus einem mit einer pharmakologisch aktiven Substanz getränkten Faden, der in dem infizierten Bereich befestigbar ist und aus dem die Substanz langsam freisetzbar ist, **dadurch gekennzeichnet, daß** der Faden (4) als Röntgenkontrastmittel (1) einen Metallfaden (1), insbesondere einen Kupfer- oder Silberfaden, enthält.

2. Mittel gemäß Anspruch 1, **dadurch gekennzeichnet, daß** der Metallfaden (1) im Zentrum des Fadens (4) angeordnet und von die Substanz freisetzendem Gewebe (3) umgeben ist.

3. Mittel gemäß Anspruch 2, **dadurch gekennzeichnet, daß** zwischen dem Metallfaden (1) und dem Gewebe (3) eine isolierende Schicht (2) angeordnet ist.

4. Mittel gemäß Anspruch 2, **dadurch gekennzeichnet, daß** das Gewebe (3) aus Poly(ethylen, vinylacetat) besteht.

5. Mittel gemäß Anspruch 1, **dadurch gekennzeichnet, daß** die pharmakologisch aktive Substanz ein Antibiotikum, ein entzündungshemmender oder ein sauerstoffabspaltender Stoff ist.

6. Mittel gemäß Anspruch 5, **dadurch gekennzeichnet, daß** das Antibiotikum Tetracyclin oder Metronidazol ist.

## Claims

1. A composition for treating topical periodontal infections, consisting of a thread saturated with a pharmacologically active substance, said thread being attachable in the infected area and being able to slowly release the substance, **characterised in that** the thread (4) contains a metal thread (1), especially a copper or silver thread, as x-ray contrast medium.

2. The composition of claim 1, **characterised in that** the metal thread (1) is located in the centre of the thread (4), and is surrounded by fabric (3) that releases the substance.

3. The composition of claim 2, **characterised in that** an insulating layer (2) is interposed between the metal thread (1) and the fabric (3).

4. The composition of claim 2, **characterised in that** the fabric (3) consists of polyethylene / polyvinyl acetate.

5. The composition of claim 1, **characterised in that** the pharmacologically active substance is an antibiotic, an antiinflammatory or a deoxygenating material.

6. The composition of claim 5, **characterised in that** the antibiotic is tetracycline or metronidazole

## Revendications

1. Moyen pour le traitement d'infections parodontales locales, composé d'un fil imprégné d'une substance pharmacologiquement active qui peut être fixé dans la zone infectée et dont la substance peut être libérée lentement, **caractérisé en ce que** le fil (4) contient comme substance de contraste aux rayons X un fil métallique (1), notamment un fil de cuivre ou d'argent.

2. Moyen selon la revendication 1, **caractérisé en ce que** le fil métallique (1) est disposé au centre d'un fil (4) et est entouré du tissu (3) libérant la substance.

3. Moyen selon la revendication 2, **caractérisé en ce qu'**une couche isolante (2) est disposée entre le fil métallique (1) et le tissu (3).

4. Moyen selon la revendication 2, **caractérisé en ce que** le tissu (3) se compose de copolymères d'éthylène-acétate de vinyle.

5. Moyen selon la revendication 1, **caractérisé en ce que** la substance pharmacologiquement active est un antibiotique, une substance anti-inflammatoire ou une substance libérant de l'oxygène.

6. Moyen selon la revendication 5, **caractérisé en ce que** l'antibiotique est de la tétracycline ou du métronidazole.
